# EUROPÄISCHE PATENTSCHRIFT

(11) **EP 3 466 480 B1**
(45) Veröffentlichungstag und Bekanntmachung des Hinweises auf die Patenterteilung: **26.02.2025**
(21) Anmeldenummer: 17195241.9
(22) Anmeldetag: 06.10.2017
(51) Int. Cl.: A61M 39/10, A61B 1/00

(54) **VERBINDUNGSVORRICHTUNG FÜR GASTRISCHE KALIBRIERSCHLÄUCHE SOWIE MEDIZINISCHES SYSTEM MIT EINER VERBINDUNGSVORRICHTUNG FÜR GASTRISCHE KALIBRIERSCHLÄUCHE UND EINEM GASTRISCHEN KALIBRIERSCHLAUCH**
CONNECTING DEVICE FOR GASTRIC CALIBRATION HOSES AND MEDICAL SYSTEM COMPRISING A CONNECTION DEVICE FOR GASTRIC CALIBRATION HOSES AND A GASTRIC CALIBRATION HOSE
DISPOSITIF DE CONNEXION POUR TUBES D'ÉTALONNAGE GASTRIQUES AINSI QUE SYSTÈME MÉDICAL DOTÉ D'UN DISPOSITIF DE CONNEXION POUR TUBES D'ÉTALONNAGE GASTRIQUES ET D'UN TUBE D'ÉTALONNAGE GASTRIQUE

(43) Veröffentlichungstag der Anmeldung: 10.04.2019
(73) Patentinhaber: Q Medical International AG, 8260 Stein am Rhein (CH)
(72) Erfinder: Desombre, Rainer, 40667 Meerbusch (DE); Eisenzimmer, Eugenius, 67661 Kaiserslautern (DE)
(74) Vertreter: dompatent von Kreisler Selting Werner - Partnerschaft von Patent- und Rechtsanwälten mbB

(56) Entgegenhaltungen:
- EP-A1- 1 629 764
- WO-A1-02/18005
- WO-A1-2009/021030
- WO-A1-2011/066586
- WO-A1-2016/158937
- US-A- 4 511 163
- US-A- 5 776 117

## Beschreibung

Die Erfindung betrifft eine Verbindungsvorrichtung für gastrische Kalibrierschläuche sowie ein medizinisches System umfassend eine Verbindungsvorrichtung sowie ein gastrischen Kalibrierschlauch.

Aus dem medizinischen Bereich sind generell Verbindungsvorrichtungen bekannt mittels deren Fluidverbindungen zwischen unterschiedlichen fluidführenden medizinischen Geräten (wie beispielsweise Spritzen, Kanülen, Kathetern, Dreiwegehähnen und sonstigen tubulären Strukturen) hergestellt werden können.

Es ist dabei bekannt, dass die Verbindungsvorrichtungen an gegenüberliegenden Enden jeweils über eine erste und zweite Einrichtung zur Ankopplung der unterschiedlichen medizinischen Geräte verfügen. Bei der Ankopplung wird eine trennbare Fluidverbindung zwischen den unterschiedlichen Geräten hergestellt. Die Abdichtung zwischen den anzukoppelnden Teilen wird dabei häufig über aneinander angepasste Kegelflächen gewährleistet.

Nachteilig an den aus dem Stand der Technik bekannten Verbindungsvorrichtungen ist, dass an die Verbindungsvorrichtungen jeweils nur ein bestimmtes medizinisches Gerät ankoppelbar ist. Folglich müssen jeweils an die Abmessungen der verwendeten medizinischen Geräte angepasste Verbindungsvorrichtungen genutzt werden.

Aus dem Stand der Technik sind weiterhin gastrische Kalibrierschläuche für die Durchführung von medizinischen laparoskopischen Operationsverfahren, wie insbesondere im Rahmen der chirurgischen Verfahren, wie beispielsweise die biliopankreatische Diversion mit Duodenal-Switch (LBPD-DS), der banded Roux-en-Y Gastric Bypass (LBRYGB), die Banded Sleeve-Gastrektomie (LBSG), die konventionelle Sleeve-Gastrektomie (LCSG), der konventionelle Roux-en-Y Gastric Bypass (LCRYGB) und der Banded gastric bypass bekannt.

Die vorgenannten gastrischen Kalibrierschläuche weisen dabei an ihrem ersten/proximalen, dem behandelten Arzt zugewandten, Schlauchende eine Verbindungsvorrichtung zur Ankopplung von medizinischen Geräten an den gastrischen Kalibrierschlauch auf. Die Verbindungsvorrichtungen sind über ihr erstes Ende dabei im Bereich des ersten/proximalen Schlauchendes mit dem gastrischen Kalibrierschlauch verbunden. Die mit den gastrischen Kalibrierschläuchen verbundenen Verbindungsvorrichtungen weisen dabei an ihrem zweiten, dem Kalibrierschlauch abgewandten Ende, eine Einrichtung zur Ankopplung von medizinischen Geräten auf. Die Ankopplungseinrichtung verfügt dabei über eine fest vorgegebene Geometrie, derart, dass lediglich medizinische Geräte ankoppelbar sind, welche die entsprechenden, zur Ankopplungseinrichtung kompatiblen Abmessungen und/oder Anschlussgeometrien aufweisen.

Nachteilig an dem vorgenannten Stand der Technik ist, dass an dem Kalibrierschlauch jeweils nur die medizinischen Geräte mit dem richtigen, fest vorgegebenen, Geometrien an den Kalibrierschlauch angeschlossen werden können.

Dabei ist es jedoch im Rahmen der mit dem gastrischen Kalibrierschlauch durchzuführenden medizinischen Operationen notwendig bzw. wünschenswert, unterschiedliche medizinische Gegenstände an diesem anzukoppeln, wie beispielsweise unterschiedliche Spritzen, Kanülen und/oder Saugschläuche mit unterschiedlichen Geometrien anzuschließen.

Problematisch ist dabei, dass die vorgenannten medizinischen Geräte alle dieselben Abmessungen im Bereich der Ankopplungseinrichtung aufweisen müssen, was beispielsweise bei unterschiedlichen Kanülen oder Spritzengrößen nur in begrenzten Umfang möglich ist, derart, dass regelmäßig weitere Adapterstücke zur Ankopplung der vorgenannten medizinischen Geräte erforderlich sind.

Der Erfindung liegt daher die Aufgabe zugrunde, eine Verbindungsvorrichtung für gastrische Kalibrierschläuche, sowie ein medizinisches System umfassend eine Verbindungsvorrichtung für Kalibrierschläuche sowie ein gastrischen Kalibrierschlauch zu schaffen, welche die zuvor aufgeführten Nachteile des Stands der Technik nicht aufweist und eine einfache und sichere Ankopplung von unterschiedlichen medizinischen Geräten mit unterschiedlichsten Anschlussgeometrien zu gewährleisten. Folgende Verbindungsvorrichtungen sind Teil des Stands der Technik: WO 2016/158937, WO 2011/066586, US 5776117, US 4511163.

Zur Lösung der Aufgabe dienen die Merkmale des Anspruchs 1 sowie des Anspruchs 6.

Erfindungsgemäß ist bei einer Verbindungsvorrichtung für gastrische Kalibrierschläuche mit einem ersten und einem gegenüberliegenden zweiten Ende, wobei am ersten Ende eine erste Anschlusseinrichtung und am zweiten Ende eine zweite Anschlusseinrichtung ausgebildet ist, wobei die erste und die zweite Anschlusseinrichtung miteinander über einen Fluidkanal in Verbindung stehen, und wobei die erste Anschlusseinrichtung zumindest eine erste sich verjüngende Vertiefung aufweist, in die zur Ankopplung gastrische Kalibrierschläuche einsteckbar sind.

Die erfindungsgemäße Verbindungsvorrichtung ist dadurch gekennzeichnet, dass die zweite Anschlusseinrichtung zur Ankopplung von unterschiedlichen medizinischen Geräten zumindest eine zweite sich verjüngende Vertiefung und zumindest eine, die zweite Vertiefung ringförmig umgebende sich verjüngende Ausnehmung aufweist, so dass die unterschiedlichen medizinischen Geräte wahlweise in die zweite sich verjüngende Vertiefung oder in die sich verjüngende Ausnehmung zum Ankoppeln einsteckbar sind.

Die Verbindungsvorrichtung kann dabei über einen im wesentlichen zylinderförmigen Körper mit einer Körperlängsachse verfügen, wobei die erste und zweite Anschlusseinrichtung an gegenüberliegenden ersten und zweiten Enden des Zylinders angeordnet sein können. In einer bevorzugten Ausführungsform kann der die Verbindungseinrichtung bildende Körper kreiszylinderförmig ausgeführt sein. Die Verbindungsvorrichtung kann dabei insbesondere eine Längsachse aufweisen und der Fluidkanal kann im Wesentlichen Parallel zu der Längsachse der Verbindungsvorrichtung verlaufen.

Bei den unterschiedlichen medizinischen Geräten kann es sich beispielsweise um Spritzen, Kanülen, Fingertips sowie tubulären Strukturen, wie beispielsweise Saugschläuche handeln. Dabei ist entscheidend, dass die unterschiedlichen medizinischen Geräte jeweils zwar über eine angeformte Anschlusseinrichtung verfügen, welche jedoch aufgrund der unterschiedlichen Abmessungen der vorgenannten medizinischen Geräte ebenfalls deutlich unterschiedliche Abmessungen und darüber hinaus auch unterschiedliche geometrischen Ausgestaltungsformen aufweisen kann.

Bei einem Fingertip handelt es sich um einen Fluidkanal mit zwei gegenüberliegenden Anschlusseinrichtungen, sowie einer seitlichen Öffnung am Fluidkanal. Bei einer Zwischenschaltung des Fingertips zwischen einer Unterdruckquelle (Absaugpumpe) und einem Absaugschlauch oder Kalibrierschlauch kann über die seitliche Öffnung des Fingertips, der Sog innerhalb des Absaugschlauches oder Kalibrierschlauches beliebig reguliert werden. Durch ein wiederholtes Freigeben und Blockieren der seitlichen Öffnung, beispielsweise mittels einer Fingerspitze, kann ein intermittierender Absaugvorgang realisiert werden.

Unter dem Begriff der Ankopplung, beziehungsweise des Ankoppelns, ist die Herstellung einer trennbaren Fluidverbindung zwischen den unterschiedlichen anzukoppelnden medizinischen Geräten und der Verbindungsvorrichtung zu verstehen.

Die erste und/oder zweite sich verjüngende Vertiefung steht an ihrem, sich in der Verbindungsvorrichtung befindlichen, und damit an ihrem endabgewandten Abschluss jeweils in Fluidverbindungen mit dem Fluidkanal. Somit kann die erste und/oder zweite sich verjüngende Vertiefung ebenfalls als Teil des Fluidkanals angesehen werden. Dabei kann die erste und/oder zweite sich verjüngende Vertiefung am Übergang zum Fluidkanal im Wesentlichen die gleichen Abmessungen wie der Fluidkanal aufweisen. In einer alternativen Ausführungsform ist es jedoch auch denkbar, dass an dem Übergang zwischen der ersten und/oder zweiten sich verjüngenden Vertiefung zu dem Fluidkanal eine Durchmesseränderung vorgesehen ist, derart, dass an dem Übergang eine Stufe ausgestaltet ist.

Die erfindungsgemäße Ausgestaltung der Verbindungsvorrichtung weist dabei den Vorteil auf, dass im Bereich der zweiten Anschlusseinrichtung unterschiedliche medizinische Geräte mit unterschiedlichen Anschlusssystemen beziehungsweise auch insbesondere unterschiedlichen Anschlussgeometrien ankoppelbar sind. Dies weist den besonderen Vorteil auf, dass der Arzt während der Nutzung eines, eine erfindungsgemäße Verbindungsvorrichtung aufweisen, gastrischen Kalibrierschlauchs auf unterschiedliche medizinische Geräte mit insbesondere unterschiedlichen Anschlussgeometrien zurückgreifen kann ohne an dem gastrischen Kalibrierschlauch die angekoppelte Verbindungsvorrichtungen austauschen zu müssen und ohne Adapter nutzen zu müssen.

Über die zweite Anschlusseinrichtung kann dabei eine Vielzahl unterschiedlicher medizinsicher Geräte mit unterschiedlichen Anschlussgeometrien und/oder - systemen angekoppelt werden, so dass eine Nutzung von zusätzlichen Adapterstücken gänzlich vermieden werden kann. Weiterhin ist es nunmehr möglich, dass der behandelnde Arzt während der Nutzung des Kalibrierschlauches auf die jeweils gewünschten medizinischen Geräte, wie beispielsweise Spritzen mit unterschiedlichen Volumina, Fingertips oder Saugschläuchen zurückgreifen kann und durch die zweite Anschlusseinrichtung dabei sichergestellt ist, das jegliche der vorgenannten medizinischen Geräte unmittelbar an die Verbindungsvorrichtung ankoppelbar sind. Die erfindungsgemäße Ausgestaltung eröffnet dadurch für das Klinikpersonal beziehungsweise den behandelnden Arzt die Möglichkeit der flexiblen Auswahl unterschiedlichster medizinischer Geräte.

Erfindungsgemäß verjüngt sich die erste Vertiefung von dem ersten Ende in Richtung des zweiten Endes und die zweite Vertiefung von dem zweiten Ende in Richtung des ersten Endes.

Die sich verjüngende Ausgestaltung der ersten und/oder zweiten Vertiefung weist dabei den Vorteil auf, dass die über die erste und/oder zweite Vertiefung die anzukoppelnden medizinischen Geräte zunächst einfach in den Bereich der ersten oder zweiten Anschlusseinrichtung einbringbar sind. Die sich verjüngende Ausgestaltung der Vertiefung weist weiterhin den Vorteil auf, dass sich bei Einbringung der medizinischen Geräte in die sich verjüngende Vertiefung das anzukoppelnde Gerät selbstständig in der Vertiefung zentriert und ausrichtet, derart, dass ein Verkanten des anzukoppelnden medizinischen Geräts weitgehend vermieden wird. Dabei kann es vorgesehen sein, dass die notwenige Dichtung zwischen der sich verjüngenden Vertiefung und dem anzukoppelnden medizinischen Gerät darüber sichergestellt ist, dass das anzukoppelnde medizinische Gerät ein an den Verlauf der sich verjüngenden Vertiefung angepassten Außenoberflächenverlauf aufweist.

Die erste sich verjüngende Vertiefung und/oder die zweite sich verjüngende Vertiefung kann einen Teil des Fluidkanals bilden.

In einer weiteren Ausgestaltungsform, kann es vorgesehen sein, dass die sich gegenüberliegende erste und zweite sich verjüngende Vertiefung an ihrem endabgewandten Abschluss ineinander nahtlos übergehen, derart, dass der eigentliche Fluidkanal der Verbindungsvorrichtung lediglich durch die sich gegenüberliegenden verjüngenden Vertiefungen ausgebildet wird. Eine derartige Ausgestaltungsform weist dabei den Vorteil auf, dass die Größe beziehungsweise insbesondere die Länge der Verbindungvorrichtung kurz gehalten werden kann. Damit können besonders kompakte Ausführungen von Verbindungsvorrichtungen erreicht werden.

Erfindungsgemäß ist zwischen der zweiten Vertiefung und der Ausnehmung eine Begrenzungswand und eine die Ausnehmung umgebende Außenwand ausgebildet, wobei die Außenwand gegenüber der Begrenzungswand in Richtung des zweiten Endes zurückgezogen ausgestaltet ist, derart, dass die Begrenzungswand das erste Ende bildet. Die vorgenannte Ausführungsform weist dabei den Vorteil auf, dass die zweite Anschlusseinrichtung zwei unterschiedliche Ankopplungseinrichtungen erhält. Die erste Ankopplungsmöglichkeit wird dabei über die sich verjüngende Vertiefung bzw. den Innenkonus gebildet. Eine weitere, von der vorgenannten unabhängige, Ankopplungsmöglichkeit wird durch die zwischen der Begrenzungs- und Außenwand eingeschlossenen Ausnehmung geschaffen.

Vorteilhafterweise kann es vorgesehen sein, dass sich ein Außendurchmesser der Begrenzungswand von dem ersten Ende in Richtung des zweiten Endes vergrößert. Durch den sich vergrößernden Außendurchmesser der Begrenzungswand kann diese als weitere separate Ankopplungsmöglichkeit für medizinische Geräte mit Innenkonus bzw. Ausnehmung genutzt werden. Weiterhin lassen sich durch den vergrößernden Außendurchmesser anzukoppelnde Gegenstände einfacher auf die Begrenzungswand aufschieben und in den Bereich der Ausnehmung hinführen.

Weiterhin ist es erfindungsgemäß vorgesehen, dass die Begrenzungswand im Bereich des zweiten Endes abgeschrägt ist.

Die Abschrägung der Begrenzungswand ist dabei derart ausgeführt, dass die Begrenzungswand eine Stirnfläche aufweist die im Hinblick auf die Längsachse der Verbindungvorrichtung schräg steht, derart, dass zwischen der schrägen Ebene der Stirnfläche und der Körperlängsachse des Verbindungseinrichtung Winkel ungleich 90 Grad eingeschlossen wird. Die Abschrägung der Begrenzungswand weist dabei den Vorteil auf, dass die an dem Ende der Begrenzungswand gebildet Öffnung durch eine ovale bzw. elliptische querstehende Stirnwandfläche begrenzt wird. Durch die querstehende ovale Stirnwandfläche wird die Einführbarkeit von medizinischen Geräten in die Öffnung bzw. durch die Begrenzungswand umschlossene Vertiefung vereinfacht.

Es kann weiterhin vorgesehen sein, dass die ringförmige Ausnehmung von dem zweiten Ende in Richtung des ersten Endes sich kontinuierlich verjüngt, wobei die Abmessungen der Ausnehmung an die durchschnittliche Wanddicke der an die zweite Anschlusseinrichtung anzukoppelnden medizinischen Geräte angepasst ist. Die vorgenannte sich kontinuierlich verjüngende Ausgestaltung der ringförmigen Ausnehmung weist dabei den Vorteil auf, dass medizinische Geräte mit schlauchförmigen Strukturen, ohne besonderes Anschlusssystem und mit unterschiedlichen Wanddicken über die zweite Anschlusseinrichtung direkt an die Verbindungseinrichtung angekoppelt werden können. Bei der Ankopplung wird die Schlauchwand in die Ausnehmung eingebracht bis die Wand zwischen der Begrenzungs- und der Außenwand eingeklemmt ist über die Klemmung wird gleichzeitig ein Abdichtung erzielt.

In einer besonders bevorzugten Ausführungsform kann es vorgesehen sein, dass die Abmessung der ersten Vertiefung an einen Außendurchmesser des anzukoppelnden gastrischen Kalibrierschlauchs angepasst ist.

Bevorzugt ist die erste Vertiefung für die Ankopplung von gastrischen Kalibrierschläuchen mit einem Außendurchmesser im Bereich von etwa 30 bis 45 Fr (French) ausgestaltet.

Nach einer weiteren bevorzugten Ausführungsform ist die Abmessung der ersten Vertiefung zur Ankopplung eines Kalibrierschlauches mit einem Außendurchmesser von 32, 34, 36, 38 oder 40 Fr (French) angepasst.

Die Angabe "French", Abkürzung "Fr" bezeichnet ein Maß für den Außendurchmesser von tubulären Strukturen, wie beispielsweise Kanülen oder Kalibrierschläuchen. Drei French entsprechen dabei genau einem Millimeter, beziehungsweise 1 Fr = 1/3 mm. Die Fr-Zahl ist also immer dreimal so groß wie der Außendurchmesser in Millimetern. Im französischen Sprachraum wird die Bezeichnung "Charriere", Abkürzung "Ch" gleichbedeutend zu der Bezeichnung "French" verwendet. Im US-amerikanischen Sprachraum wird für den Außendurchmesser der tubulären Strukturen ebenfalls die Maßangabe "Gauge", Abkürzung "G" verwendet, welche jedoch nicht den Maßangaben in Charrière oder French entspricht.

Der nachfolgend eingeblendeten Tabelle kann der grundsätzliche Zusammenhang zwischen den unterschiedlichen Maßskalen entnommen werden:

| French [ ] | Gauge [ ] | Außendurchmesser [mm] |
|---|---|---|
| 1 | 29 | 0,33 |
| 2 | 23 | 0,635 |
| 3 | 19 | 1,067 |
| 4 | 18 | 1,27 |
| 5 | 16 | 1,651 |
| 10 | 10 | 3,404 |

Bei einer kreiszylinderförmigen Ausgestaltung der Verbindungsvorrichtung kann die erste Vertiefung als Innenkonus ausgeführt werden, wobei der Innenkonus im Bereich des zweiten Endes der Verbindungsvorrichtung über einen maximalen Öffnungsdurchmesser verfügt, und sich im Verlauf in Richtung des ersten Endes bis zu einem zweiten Minimaldurchmesser verjüngt.

Es kann vorgesehen sein, dass die erste Vertiefung von dem ersten Ende in Richtung des zweiten Endes mindestens zwei sich aneinander anschließende, sich jeweils verjüngende Stufen zur Aufnahme von gastrischen Kalibrierschläuchen mit unterschiedlichen mittleren Durchmessern umfasst, wobei die Stufen unterschiedliche Anfangs- und Enddurchmesser aufweisen und wobei sich der jeweilige mittlere Durchmesser der Stufen von dem ersten Ende in Richtung des zweiten Endes jeweils kontinuierlich reduziert.

Die stufenförmige Ausgestaltung der ersten Vertiefung mit jeweils unterschiedlichen mittleren Durchmessern ermöglicht es über die unterschiedlichen Stufen der ersten Vertiefung unterschiedliche gastrische Kalibrierschläuche mit unterschiedlichen mittleren Außendurchmessern anzukoppeln.

In einer weiteren bevorzugten Ausführungsform kann es vorgesehen sein, dass die erste Vertiefung am ersten Ende eine abgeschrägte Fase umfasst.

Die Fase weist dabei von dem zweiten Ende in Richtung des ersten Endes eine von außen nach innen geneigte Oberfläche auf. Die Fase weist dabei den Vorteil auf, dass die über die erste Vertiefung anzukoppelnden gastrischen Kalibrierschläuche in die erste Vertiefung vereinfacht eingeführt werden können. Durch die Fase wird der Kalibrierschlauch beim Einführen in die erste Vertiefung zentriert, wodurch eine Verkeilung oder eine Schrägstellung des Kalibrierschlauchs bei dem Einführen in die Vertiefung verhindert wird.

Es kann weiterhin vorgesehen sein, dass im Bereich der Öffnungsdurchmesser der sich verjüngenden Stufen der ersten Vertiefung jeweils Fasen angeordnet werden.

Erfindungsgemäß umfasst ein medizinisches System eine Verbindungsvorrichtung für Kalibrierschläuche, sowie einen gastrischen Kalibrierschlauch, wobei der Kalibrierschlauch zumindest eine Schlauchlängsachse, ein erstes und ein gegenüberliegendes zweites Schlauchende und eine Schlauwandung aufweist, die einen Hohlraum umschließt, wobei am zweiten Schlauchende ein den Hohlraum begrenzendes Abschlusselement angeordnet ist, wobei das Abschlusselement mindestens ein sich im Wesentlichen parallel zu der Schlauchlängsachse erstreckenden durch das Abschlusselement verlaufenden Durchlass aufweist, wobei das erste Schlauchende über die erste Anschlusseinrichtung an die Verbindungsvorrichtung angekoppelt ist.

Erfindungsgemäß kann ein gastrischer Kalibrierschlauch zumindest eine Schlauchlängsachse, ein erstes und ein gegenüberliegendes zweites Schlauchende und eine Schlauwandung aufweisen, wobei die Schlauchwandung einen Hohlraum umschließt, wobei am zweiten Schlauchende ein den Hohlraum begrenzendes Abschlusselement angeordnet ist, wobei das Abschlusselement mindestens ein sich im Wesentlichen parallel zu der Schlauchlängsachse erstreckenden durch das Abschlusselement verlaufenden Durchlass und/oder eine Öffnung aufweist.

Das erste Schlauchende kann dabei im Wesentlichen durch eine Schlauchöffnung gebildet sein, die ihrerseits lediglich durch das Ende der Schlauchwanderung ausgebildet ist, derart, dass am ersten Ende der Hohlraum ausgebildet ist.

Der gastrische Kalibierschlauch kann bevorzugt eine Länge im Bereich von etwa 1.000 mm bis 1.300mm aufweisen.

Das vorgenannte medizinische System weist dabei den Vorteil auf, dass an dem gastrischen Kalibrierschlauch eine Vielzahl von unterschiedlichen medizinischen Geräten mit unterschiedlichen Geometrien ankoppelbar ist.

In einer vorteilhaften Ausführungsform kann es vorgesehen sein, dass die Schlauchwandung im Bereich des zweiten Schlauchendes mehrere Öffnungen umfasst. Durch die mehreren Öffnungen wird die Ausbildung einer Verstopfung bei dem Erzeugen eines Unterdrucks in dem Hohlraum des Kalibreischlauches reduziert bzw. kann gänzlich verhindert werden. Durch die Vielzahl der Öffnungen wird der anliegende Unterdruck auf eine Vielzahl von Öffnungen verteilt, derart, dass die auf ein eventuell angesaugtes Körpergewebe im Bereich der Öffnungen wirkende Saugkraft und damit das Verletzungsrisiko deutlich reduziert werden kann.

Bevorzugt kann die Schlauchwandung des gastrischen Kalibrierschlauches einen Außendurchmesser im Bereich von etwa 30 bis 45 Fr (French) aufweisen.

Nach einer weiteren bevorzugten Ausführungsform weist der Kalibrierschlauch eine Schlauchwandung mit einem Außendurchmesser von 32, 34, 36, 38 oder 40 Fr (French) auf. Die Schlauchwandung des gastrischen Kalibrierschlauches kann mit einem Farbstreifen entlang der Schlauchlängsachse versehen sein, wobei die Farbe des Streifens sich für unterschiedliche Außendurchmesser unterscheidet. Die farbliche Codierung weist den Vorteil auf, dass Größenverwechslungen der Kalibrierschläuche vermieden werden können.

Die Schlauchwandung kann entlang der Längsachse mit Längenmarkierungen versehen sein. Die Längenmarkierungen ermöglichen dem behandelnden Arzt bzw. dem Anästhesisten zu erkennen, welcher Längenanteil des Schlauches bereits in den Körper des zu behandelnden Patienten eingeführt wurde.

Es kann vorgesehen sein, dass das Abschlusselement an dessen schlauchabgewandten Ende eine abgerundete und/oder halbkugelförmige ausgeformte Fläche umfasst. Die abgerundete und/oder halbkugelförmige ausgestaltete Oberfläche weist dabei den Vorteil auf, dass bei Einführung des gastrischen Kalibrierschlauchs sich dieser dem Verlauf der Körperöffnungen einfacher anpasst und ein Umknicken oder ein in Fachkreisen sogenanntes "kinking" weitgehend verhindert wird. Weiterhin kann durch die Halbkugelförmig ausgeformte Oberfläche eine Verletzung des körpereigenen Gewebes bei einem Auftreffen und/oder Anstoßen des Abschlusselements auf eine Körperwandung weitgehend vermieden werden.

Weiterhin bevorzugt kann vorgesehen sein, dass das Abschlusselement aus einem flexiblen Kunststoffmaterial, wie beispielsweise Silikon hergestellt ist.

Das Abschlusselement kann dabei aus einem Material gefertigt werden, dass eine geringere Steifigkeit als die Schlauchwandung des gastrischen Kalibrierschlauchs aufweist. Insbesondere kann es auch vorgesehen sein, dass das Abschlusselement eine geringere Shore-Härte als die Schlauchwandung des gastrischen Kalibrierschlauchs aufweist.

Die Herstellung des Abschlusselements aus einem flexiblen Kunststoffmaterial weist dabei den Vorteil auf, dass bei einem Vorschieben des gastrischen Kalibrierschlauchs und damit bei einer Vorwärtsbewegung des Abschlusselements in den körpereigenen Hohlräumen bei dem Auftreffen und/oder Anstoßen des Abschlusselementes auf eine Körperwand eine Verletzung der Körperwand weitgehend vermieden wird. Die Ausgestaltung des Abschlusselementes aus dem flexiblen Kunststoffmaterial weist weiterhin den Vorteil auf, dass das Abschlusselement als eine Art Puffer für den gastrischen Kalibrierschlauch funktioniert. Im Falles eines Anstoßens des Abschlusselements auf ein körpereigenes Gewebe nimmt das Abschlusselement die kinetische Energie des gastrischen Kalibrierschlauchs durch eine Verformung des flexiblen Abschlusselements auf.

In einer weiteren bevorzugten Ausführungsform kann es vorgesehen sein, dass das Abschlusselement zumindest teilweise ein radiopakes Material umfasst. Die Ausgestaltung des Abschlusselements mit einem radiopaken Material weist den Vorteil auf, dass die Lage des gastrischen Kalibrierschlauches im Körperinneren mittels bildgebender Röntgenverfahren eindeutig identifizierbar ist. Der behandelnde Arzt kann dann eindeutig feststellen, ob der gastrische Kalibrierschlauch in die gewünschte Position im Körper eingebracht wurde. Das Abschlusselement kann beispielsweise Bariumsulfat enthalten.

Weiterhin kann es vorgesehen sein, dass das Abschlusselement zumindest teilweise blau eingefärbt ist.

Das blau eingefärbte Abschlusselement kann mittels minimalinvasiver bildgebender Verfahren, wie beispielsweise der Videoendoskopie, eindeutig im Körper des zu behandelnden Patienten identifiziert werden, da in dem menschlichen Körper keine blaugefärbten Gegenstände existieren.

In einer besonders bevorzugten Ausführungsform kann es vorgesehen sein, dass der gastrische Kalibrierschlauch mindestens einen Zusatzkanal umfasst, wobei der mindestens eine Zusatzkanal ein Volumen umschließt und im wesentlichen parallel zu der Schlauchlängsachse verläuft, wobei ein erstes Ende des mindestens ein Zusatzkanals in dem Bereich des ersten Schlauchendes an der äußeren Schlauchwandung endet und wobei einem zweites Ende des mindestens ein Zusatzkanals in dem Abschlusselement mündet.

Das durch den mindestens einen Zusatzkanal umschlossene Volumen ist dabei zu dem Hohlraum des gastrischen Kalibrierschlauchs komplett abgedichtet, derart, dass zwischen dem Volumen des Zusatzkanals und dem Hohlraum des gastrischen Kalibrierschlauchs keine Fluidverbindung besteht.

Es kann dabei vorgesehen sein, dass der mindestens eine Zusatzkanal als Zusatzschlauch inner- und/oder außerhalb des Kalibrierschlauchs angeordnet ist. Insbesondere kann es vorgesehen sein, dass der Zusatzkanal im Bereich der Innenwandung des Kalibrierschlauchs angeordnet ist. In einer besonders bevorzugten Ausführungsform kann es vorgesehen sein, dass der zumindest eine Zusatzkanal einstückig mit der Schlauchwandung des Kalibrierschlauchs ausgebildet ist, derart, dass eine Unterteilung des Hohlraums des Kalibrierschlauchs in mehrere Hohlräume durch Einziehung von mehreren Unterteilungswenden in den Kalibrierschlauch vorgenommen wird.

Es kann vorgesehen sein, dass in mindestens einem Zusatzkanal eine Lichtleiteinrichtung angeordnet ist, wobei im Bereich des ersten Schlauchendes an der Lichtleiteinrichtung eine Lichteintrittseinrichtung ausgestaltet ist und wobei die Lichtleiteinrichtung im zweiten Schlauchende durch das Anschlusselement zu dem schlauchabgewandten Ende verläuft, wo eine Lichtaustrittseinrichtung angeordnet ist.

Die Vorsehung der Lichtleiteinrichtung bietet dabei den Vorteil, dass über die Leiteinrichtung Lichtwellen in den Bereich des Abschlusselements geleitet werden können, derart, dass der Bereich vor dem Abschlusselement des gastrischen Kalibrierschlauchs beleuchtet werden kann, wobei die Lichtstrahlen von der Lichtaustrittseinrichtung im Bereich des Abschlusselements ausgesendet werden.

Im Folgenden wird unter Bezugnahme auf die Zeichnungen unterschiedliche Ausführungsbeispiele der Erfindung näher erläutert.

Es zeigen:

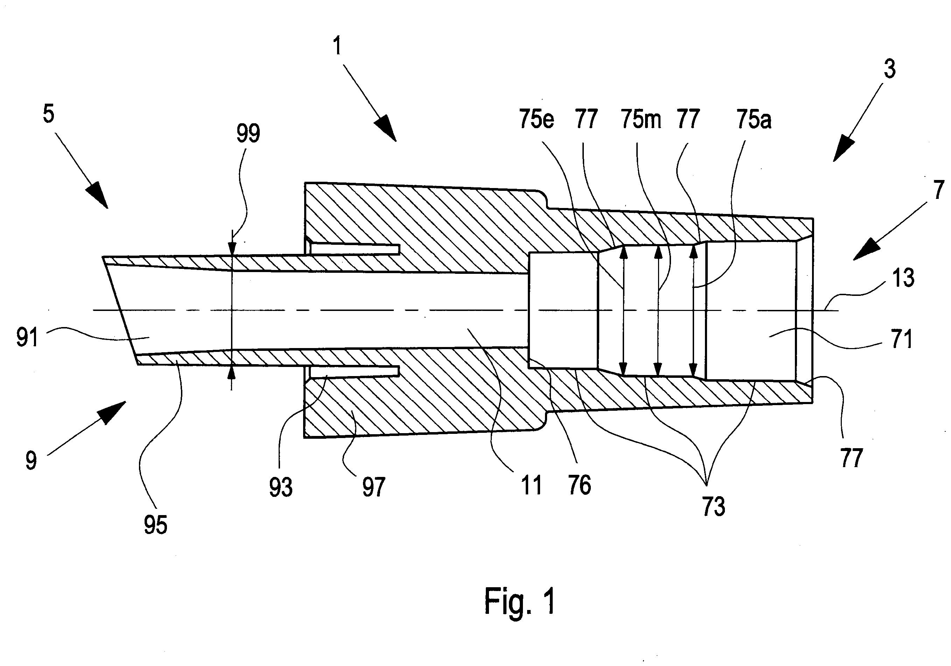

- Figur 1:: eine Schnittdarstellung eines Ausführungsbeispiels der Verbindungsvorrichtung für gastrische Kalibrierschläuche;
- Figur 2:: ein Längsschnitt durch eine Verbindungsvorrichtung mit angekoppelter großer Spritze;
- Figur 2b:: eine Schnittdarstellung einer Verbindungseinrichtung mit angekoppelter kleiner Spritze;
- Figur 2c:: eine Schnittdarstellung einer Verbindungseinrichtung mit angekoppeltem Fingertip;
- Figur 3:: eine Schnittdarstellung eines medizinischen Systems umfassend einen an einer Verbindungsvorrichtung angekoppelten gastrischen Kalibrierschlauch;
- Figur 4:: eine Schnittdarstellung eines weiteren Ausführungsbeispiels eines gastrischen Kalibrierschlauchs.

Die dargestellte Verbindungsvorrichtung (1) für gastrische Kalibrierschläuche (2) umfasst ein erstes Ende (3) und ein gegenüberliegendes zweites Ende (5), wobei an dem ersten Ende (3) eine erste Anschlusseinrichtung (7) und an dem zweiten Ende (5) eine zweite Anschlusseinrichtung (9) ausgebildet ist. Die erste und die zweite Anschlusseinrichtung (7,9) stehen dabei miteinander über einen Fluidkanal (11) in Verbindung. Die erste Anschlusseinrichtung (7) weist eine erste sich verjüngende Vertiefung (71) auf, in welche zur fluidalen Ankopplung gastrische Kalibrierschläuche (2) einsteckbar sind. Weiterhin verfügt das dargestellte Ausführungsbeispiel einer Verbindungsvorrichtung (1) über eine zweite Anschlusseinrichtung (9) zur Ankopplung von unterschiedlichen medizinischen Geräten (4), welche eine zweite sich verjüngende Vertiefung (91) und eine die zweite Vertiefung (91) ringförmig umgebende sich verjüngende Ausnehmung (93) aufweist. In die zweite sich verjüngende Vertiefung (91) oder alternativ in die sich verjüngende Ausnehmung (93) können wahlweise unterschiedliche medizinische Geräte (4) zur fluidalen Ankopplung eingesteckt und damit angekoppelt werden.

In der dargestellten Ausführungsform ist die Verbindungsvorrichtung zylinderförmig beziehungsweise kreiszylinderförmig ausgestaltet, mit einer Längsachse (13) der Verbindungsvorrichtung (1).

In dem dargestellten Ausführungsbeispiel verjüngt sich die erste Vertiefung (71) von dem Bereich des ersten Endes (3) in Richtung des zweiten Endes (5) und die zweite Vertiefung (91) verjüngt sich von dem Bereich des zweiten Endes (5) in Richtung des ersten Endes (3). Weiterhin sind die erste sich verjüngende Vertiefung (71) und die zweite sich verjüngende Vertiefung (91) in der dargestellten Ausführung der Verbindungsvorrichtung (1) derart ausgeführt, dass diese jeweils einen Teil des Fluidkanals (11) bilden. Insbesondere mündet die zweite sich verjüngende Vertiefung (91) direkt in die erste sich verjüngende Vertiefung (71), wobei eine Stufe (76) aufgrund der unterschiedlichen Durchmesser ausgebildet ist.

In dem dargestellten Ausführungsbeispiel ist zwischen der zweiten Vertiefung (91) und der Ausnehmung (93) eine Begrenzungswand (95) und eine die Ausnehmung (93) umgebende Außenwand (97) ausgebildet. Die Außenwand (97) kann dabei wie in dem dargestellten Ausführungsbeispiel gezeigt gegenüber der Begrenzungswand (95) in Richtung des ersten Endes (3) zurückgezogen ausgestaltet werden, derart, dass die Begrenzungswand (95) das zweite Ende (5) der Verbindungsvorrichtung (1) bildet.

Weiterhin ist es in der dargestellten Ausführungsform der Verbindungsvorrichtung (1) vorgesehen, dass sich der Außendurchmesser (99) der Begrenzungswand (95) von dem Bereich des zweiten Endes (5) in Richtung des ersten Endes (3) kontinuierlich vergrößert. Weiterhin ist die Begrenzungswand (95) im Bereich des zweiten Endes (5) gegenüber der Längsachse (13) abgeschrägt ausgeführt. Durch die abgeschrägte Ausführungsform der Begrenzungswand (95) wird im Bereich des zweite Endes (5) eine ovale Öffnung für die zweite Verbindungsvorrichtung (9) ausgebildet, derart dass über die Vertiefung (91) anzukoppelnde medizinische Geräte (4) vereinfacht in die Vertiefung (91) eingeführt werden können.

Die ringförmige Ausnehmung (93) ist von dem Bereich des zweiten Endes (5) in Richtung des ersten Endes sich kontinuierlich verjüngend ausgestaltet. Die erste Vertiefung (71) umfasst von dem Bereich des ersten Endes (3) in Richtung des zweiten Endes (5), drei sich aneinander anschließende jeweils verjüngende Stufen (73) zur Aufnahme von gastrischen Kalibrierschläuchen (2) mit unterschiedlichen mittleren Durchmessern (75m). Die dargestellten drei Stufen (73) weisen dabei jeweils unterschiedliche Anfangsdurchmesser (75a) und Enddurchmesser (75e) auf. Der jeweilige mittlere Durchmesser (75m) der drei Stufen (73) reduziert sich von dem ersten Ende (3) in Richtung des zweiten Endes (5) schrittweise.

Die dargestellte erste Vertiefung (7) weist im Bereich des ersten Endes (3) eine abgeschrägte Fase (77) auf, weiterhin sind an den beiden Übergängen der drei Stufen (73) ebenfalls zwei abgeschrägte Fasen (77) ausgestaltet.

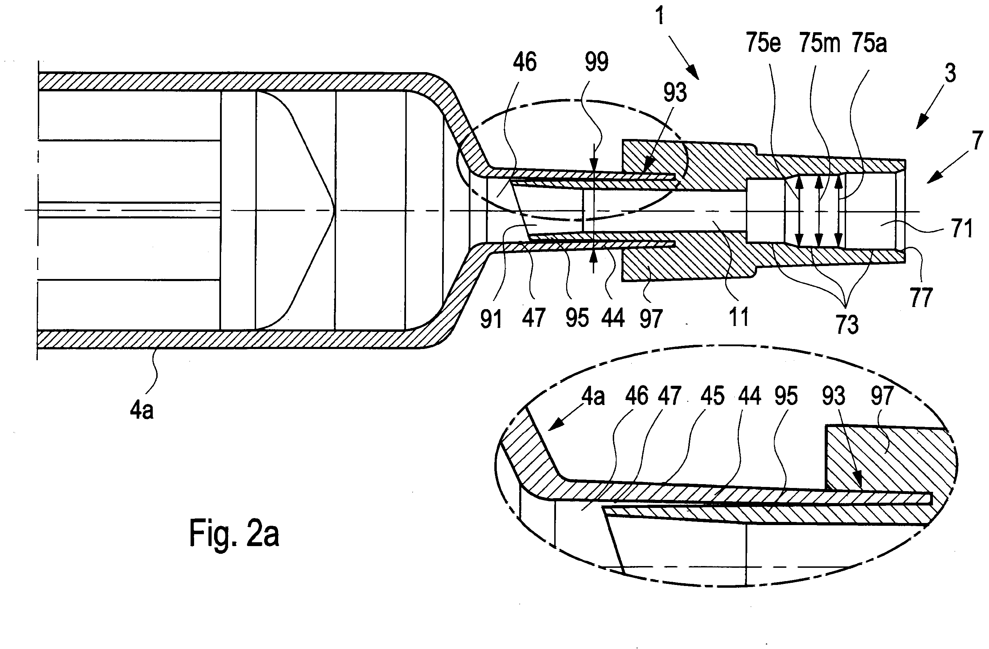

In der Figur 2a ist die Verbindungsvorrichtung (1) mit einer über die sich verjüngende Ausnehmung (93) der zweiten Anschlusseinrichtung (9) angekoppelten Spritzenvorrichtung (4a) als Beispiel eines anzukoppelnden medizinischen Gerätes (4) dargestellt. Wie dies der Figur 2a entnehmbar ist, weist das anzukoppelnde medizinische Gerät (4), beziehungsweise insbesondere die gezeigte Spritzenvorrichtung (4a), derartige Abmessungen auf, dass die Seitenwandung des Anschlusskonus (44) der Spritzenvorrichtung (4a) in der Ausnehmung (93) der Verbindungsvorrichtung (1) aufgenommen wird. Zur Ankopplung der Spritzenvorrichtung (4a) an die Verbindungsvorrichtung (1) wird zunächst die Begrenzungswand (95) in die Spritzenöffnung (46) eingebracht. Darauffolgend wird die Spritzenvorrichtung (4a) im Wesentlichen entlang der Längsachse (13) der Verbindungsvorrichtung (1) und die Verbindungsvorrichtung (13) aufeinander zubewegt, derart, dass die Außenwandung (45) des Anschlusskonus (44) zunächst in Anlage an die die Ausnehmung (93) umgebende Außenwand (97) gelangt. Dabei kommt es am zweiten Ende (5) der Begrenzungswand (95) nicht zu einer Anlage der Innenwandung (47) des Anschlusskonus (44) der Spritzenvorrichtung (4a) und der Begrenzungswand (95). Erst im weiteren Verlauf der Außenwandung (95) in Richtung auf das erste Ende (3) vergrößert sich der Außendurchmesser (99) der Außenwandung (95), derart dass es in Bereich der ringförmigen Ausnehmung (93) zu einer Anlage der Innenwandung (47) an der Begrenzungswand (95).

Die Wandung des Anschlusskonus (44) der Spritzenvorrichtung (4a) kann, wie in der Fig. 2a dargestellt, bevorzugt in der sich verjüngenden Vertiefung (93) zwischen der Begrenzungswand (95) und der Außenwand (97) eingeklemmt werden. Durch die beidseitige Klemmung des vorderen Bereiches des Anschlusskonus (44) kann dieser lokal elastisch verformt werden, wodurch eine zusätzliche Abdichtung zwischen der Spritzenvorrichtung (4a) und der Verbindungsvorrichtung (1) erzielt werden kann.

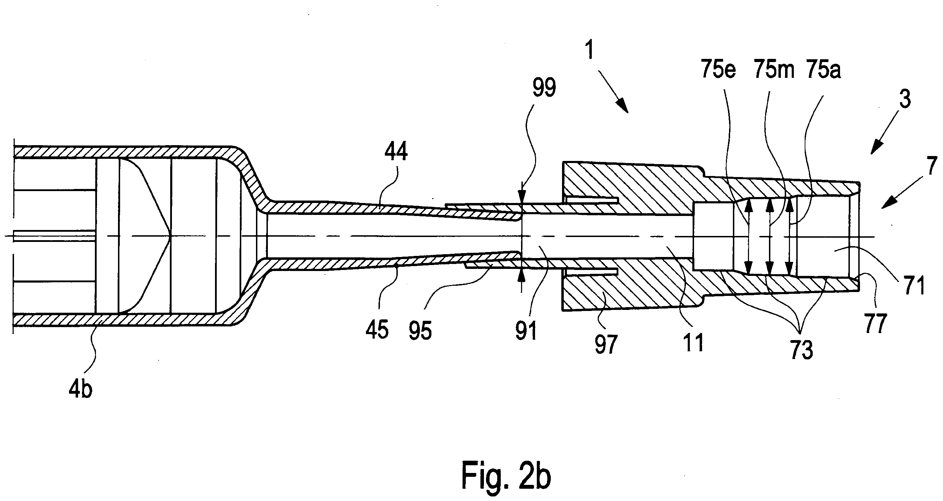

In der Figur 2b ist die Verbindungsvorrichtung (1) mit einer über die verjüngende Vertiefung (91) angekoppelten zweiten, im Vergleich zu der ,Fig. 2a kleineren Spritzenvorrichtung (4b) dargestellt. Die zweite Spritzenvorrichtung (4b) weist gegenüber der ersten Spritzenvorrichtung (4a) deutlich geringere Abmessungen, insbesondere im Bereich des Anschlusskonus (44) auf. Zur Ankopplung der Spritzenvorrichtung (4b) an die Verbindungseinrichtung (1) wird der Anschlusskonus (44) der Spritzenvorrichtung (4b) in die zweite sich verjüngende Vertiefung (91) eingeführt, wobei es schließlich zu einer Anlage der Außenwandung (45) mit der, die erste verjüngende Vertiefung (71), begrenzenden Begrenzungswand (95) kommt. Durch die vorgenannte Anlage der Außenwandung (45) an der Innenwandung der Begrenzungswand (95) wird eine Abdichtung zwischen der Spritzenvorrichtung (4b) und der Verbindungsvorrichtung (1) sichergestellt.

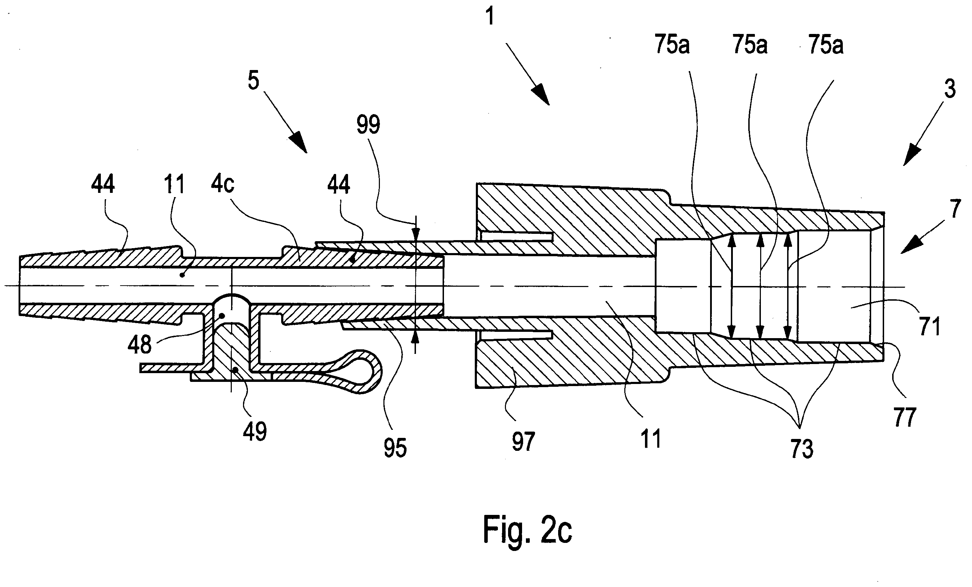

Wie dies der Figur 2c entnehmbar ist, ist es über die erste sich verjüngende Vertiefung (71) ebenfalls möglich einen sogenannten Fingertip (4c) an die Verbindungsvorrichtung (1) anzukoppeln. Der Fingertip (4c) weist einen Fluidkanal (11) mit zwei sich gegenüberliegenden Anschlusskonen (44), sowie eine seitliche Öffnung (48) des Fluidkanals (11) auf. Die seitliche Öffnung (48) ist in der Figur 2c mittels eines Verschlusses (49) abgedichtet. Der Fingertip (4c) ist über den in der Fig. 2c dargestellten rechten Anschlusskonus (44) über die zweite sich verjüngende Vertiefung (91) an die Verbindungsvorrichtung (1) abgekoppelt. Die Ankopplung erfolgt dabei sinngemäß, wie bereits im Hinblick auf Fig. 2b beschrieben.

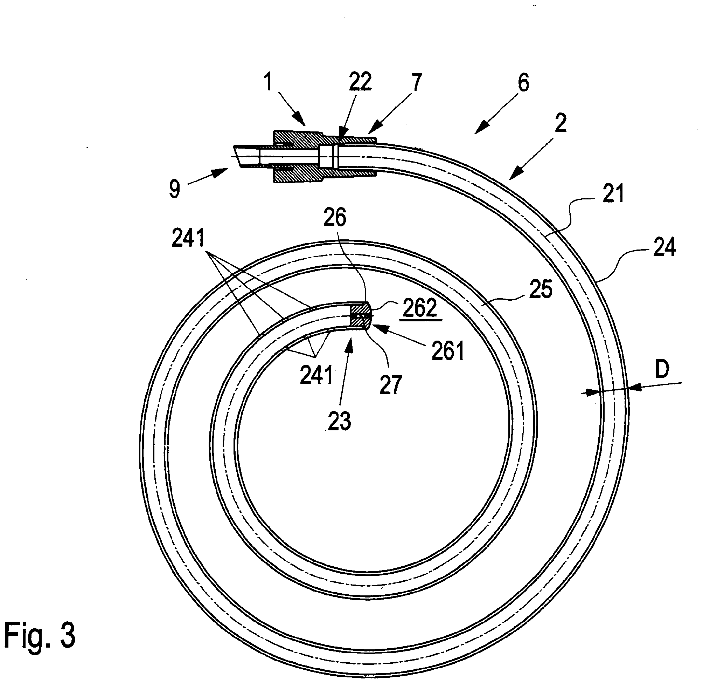

In der Figur 3 ist ein medizinisches System (6) dargestellt, welches eine Verbindungsvorrichtung (1) sowie ein gastrischen Kalibrierschlauch (2) umfasst. Der gastrische Kalibrierschlauch (2) weist eine Schlauchlängsachse (21), ein erstes und gegenüberliegendes zweites Schlauchende (22,23) und eine Schlauchwandung (24) auf, die einen Hohlraum (25) umschließt. Am zweiten Schlauchende (23) ist ein den Hohlraum (25) begrenzendes Abschlusselement (26) angeordnet, wobei das Abschlusselement (26) einen sich im Wesentlichen parallel zu der Schlauchlängsachse (21) erstreckenden durch das Abschlusselement (26) verlaufenden Durchlass (27) umfasst. Das erste Schlauchende (22) des Kalibrierschlauches (1) ist über die erste Anschlusseinrichtung (7) an die Verbindungseinrichtung (1) angekoppelt. In dem dargestellten Ausführungsbeispiel umfasst die Schlauchwandung (24) des Kalibrierschlauches (2) im Bereich des zweiten Schlauchendes (23) mehrere Öffnungen (241).

Das Abschlusselement (26) weist an dessen schlauchabgewandten Ende (261) eine abgerundete Oberfläche (262) auf.

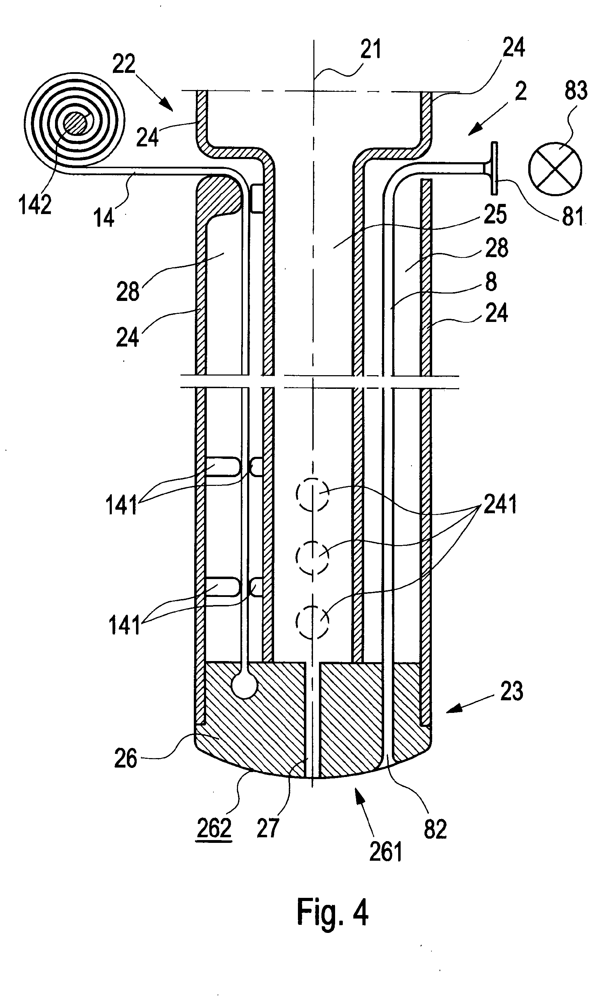

Fig. 4 zeigt eine Prinzipskizze eines weiteren Ausführungsbeispiels eines gastrischen Kalibrierschlauches (2) in Schnittdarstellung. Der gastrisch Kalibrierschlauch (2) ist grundsätzlich gebildet durch eine Schlauchwandung (24) mit einem ersten Schlauchende (22) und einem gegenüberliegenden zweitem Schlauchende (23), an dem ein Abschlusselement (26) befestigt ist. Die Schlauchwandung (24) begrenzt zusammen mit dem Abschlusselement (26) einen Hohlraum (25), wobei der Hohlraum (25) über die drei Öffnungen (241) und den im Abschlusselement (26) ausgeformten Durchlass (27) in Fluidverbindung mit dessen Umgebung steht. Am ersten Schlauchende (22) ist die Schlauchwandung (24) derart ausgestaltet, dass eine Verbindungsvorrichtung (1) (nicht dargestellt) angekoppelt werden kann, derart, dass der Kalibrierschlauch (2) zusammen mit der Verbindungsvorrichtung (1) ein medizinisches System (1) ausbilden kann. Die Schlauchwandung (24) ist in dem dargestellten Ausführungsbeispiel bis auf den Bereich des ersten Schlauchendes (22) doppelwandig ausgeführt, so dass zwei Zusatzkanäle (28) ausgebildet werden, wobei die beiden Zusatzkanäle im Bereich des zweiten Schlauchendes (23) jeweils durch das Abschlusselement (26) abgeschlossen sind und im Bereich des ersten Schlauchendes (22) durch Öffnungen über die Schlauchwandung (24) nach außen geführt sind. Es besteht somit keine Fluidverbindung zwischen den Zusatzkanälen (28) und dem Hohlraum (25).

In dem in der Figur 4 links dargestellten ersten Zusatzkanal (28) ist eine Seilzugeinrichtung (14) gegenüber der Schlauchwandung (24) über mehrere Seilzugführungen (141) längsverschieblich geführt und im Bereich des Abschlusselements (26) mit diesem verbunden. Die Seilzugeinrichtung (14) ist im Bereich des ersten Schlauchendes (22) über eine Öffnung in der Schlauchwandung (24) nach außen geführt, wo eine Aufspuleinrichtung (142) angeordnet ist. Mittels der Aufspuleinrichtung (142) ist es möglich die Seilzugeinrichtung (14) aufzuwickeln und damit den innerhalb des Zusatzkanals (28) befindlichen Anteil der Seilzugeinrichtung (14) zu verkürzen. Aufgrund der Flexibilität der Schlauchwandung (24) krümmt sich der Schlauch (2), wenn die Seilzugseinrichtung (14) auf die Aufspuleinrichtung (142) aufgewickelt wird. In dem zweiten Zusatzkanal (28) ist eine Lichtleiteinrichtung (8) geführt, welche sich im Bereich des zweiten Schlauchendes (23) durch das Abschlusselement (26) erstreckt, wobei an dem schlauchabgewandten Ende (261) des Abschlusselements (26) eine Lichtaustritteinrichtung (82) ausgebildet ist. Das gegenüberliegende Ende der Lichtleiteinrichtung (8) ist wiederum im Bereich des ersten Schlauchendes (22) über die Schlauchwandung (24) nach außen geführt, wo an der Lichtleiteinrichtung (8) eine Lichteintrittseinrichtung (81) angeordnet ist. Mittels einer Lichtquelle (83) können über die Lichteintrittseinrichtung (81) Lichtwellen in die Lichtleiteinrichtung (8) eingekoppelt und entlang der Schlauchlängsachse (21), durch den Zusatzkanal (28) und durch das Abschlusselement (26), zu der Lichtaustrittseinrichtung (82) geführt werden. An der Lichtaustrittseinrichtung (82) werden die Lichtwellen ausgekoppelt und beleuchten den Bereich vor dem zweiten Schlauchende (23).

## Patentansprüche

1. Verbindungsvorrichtung (1) für gastrische Kalibrierschläuche (2) mit einem ersten und einem gegenüberliegenden zweiten Ende (3,5), wobei am ersten Ende (3) eine erste Anschlusseinrichtung (7) und am zweiten Ende (5) eine zweite Anschlusseinrichtung (9) ausgebildet ist, wobei die erste und zweite Anschlusseinrichtung (7,9) miteinander über einen Fluidkanal (11) in Verbindung stehen und wobei die erste Anschlusseinrichtung (7) zumindest eine erste sich verjüngende Vertiefung (71) aufweist, in die zur Ankopplung gastrische Kalibrierschläuche (2) einsteckbar sind,
**dadurch gekennzeichnet,**
**dass** die zweite Anschlusseinrichtung (9) zur Ankopplung von unterschiedlichen medizinischen Geräten (4) zumindest eine zweite sich verjüngende Vertiefung (91) und zumindest eine, die zweite Vertiefung (91) ringförmig umgebende sich verjüngende Ausnehmung (93) aufweist, so dass die unterschiedlichen medizinischen Geräte (4) wahlweise in die zweite sich verjüngende Vertiefung (91) oder die sich verjüngende Ausnehmung (93) zum Ankoppeln einsteckbar sind,
wobei zwischen der zweiten Vertiefung (91) und der Ausnehmung (93) eine Begrenzungswand (95), und eine die Ausnehmung (93) umgebende Außenwand (97) ausgebildet ist, wobei die Außenwand (97) gegenüber der Begrenzungswand (95) in Richtung des ersten Endes (3) zurückgezogen ausgestaltet ist, derart, dass die Begrenzungswand (95) das zweite Ende (5) bildet,
wobei die Begrenzungswand (95) im Bereich des zweiten Endes (5) gegenüber der Längsachse (13) abgeschrägt ausgeführt ist.

2. Verbindungsvorrichtung (1) nach Anspruch 1, **dadurch gekennzeichnet, dass** sich die erste Vertiefung (71) von dem ersten Ende (3) in Richtung des zweiten Endes (5) und die zweite Vertiefung (91) von dem zweiten Ende (5) in Richtung des ersten Endes (3) verjüngt.

3. Verbindungsvorrichtung (1) nach Anspruch 1 oder 2, **dadurch gekennzeichnet, dass** die erste sich verjüngende Vertiefung (71) und/oder die zweite sich verjüngende Vertiefung (91) einen Teil des Fluidkanals (11) bilden.

4. Verbindungsvorrichtung (1) nach Anspruch 3, **dadurch gekennzeichnet, dass** sich ein Außendurchmesser (99) der Begrenzungswand (95) von dem ersten Ende (3) in Richtung des zweiten Endes (5) verkleinert.

5. Verbindungsvorrichtung (1) nach einem der Ansprüche 1 bis 4, **dadurch gekennzeichnet, dass** die erste Vertiefung (71) von dem ersten Ende (3) in Richtung des zweiten Endes (5) mindestens zwei sich aneinander anschließende, jeweils verjüngende Stufen (73) zur Aufnahme von gastrischen Kalibrierschläuchen (2) mit unterschiedlichen mittleren Durchmessern (75m) umfasst, wobei die Stufen (73) unterschiedliche Anfangs- und Enddurchmesser (75a,75e) aufweisen und wobei sich der jeweilige mittlere Durchmesser (75m) der Stufen (73) von dem ersten Ende (3) in Richtung des zweiten Endes (5) jeweils kontinuierlich reduziert.

6. Medizinisches System (6) umfassend eine Verbindungsvorrichtung (1) nach einem der Ansprüche 1 bis 5, sowie einen gastrischen Kalibrierschlauch (2), wobei der Kalibrierschlauch (2)
- zumindest eine Schlauchlängsachse (21),
- ein erstes und ein gegenüberliegendes zweites Schlauchende (22,23), und
- eine Schlauchwandung (24) aufweist, die einen Hohlraum (25) umschließt,
- wobei am zweiten Schlauchende (23) ein den Hohlraum (25) begrenzendes Abschlusselement (26) angeordnet ist, wobei das Abschlusselement (26) mindestens einen sich im Wesentlichen parallel zu der Schlauchlängsachse (21) erstreckenden durch das Abschlusselement (26) verlaufenden Durchlass (27) aufweist,
wobei das erste Schlauchende (22) über die erste Anschlusseinrichtung (7) an die Verbindungsvorrichtung (1) angekoppelt ist.

7. Medizinisches System (6) nach Anspruch 6, **dadurch gekennzeichnet, dass** die Schlauchwandung (24) im Bereich des zweiten Schlauchendes (23) mehrere Öffnungen (241) umfasst.

8. Medizinisches System (6) nach Anspruch 6 oder 7, **dadurch gekennzeichnet, dass** der Kalibrierschlauch (2) mindestens einen Zusatzkanal (28) umfasst, wobei der mindestens eine Zusatzkanal (28) ein Volumen (281) umschließt und im Wesentlichen parallel zu der Schlauchlängsachse (21) verläuft, wobei ein zweites Ende (283) des mindestens einen Zusatzkanals (28) in dem Abschlusselement (26) mündet.

9. Medizinisches System (6) nach Anspruch 8, **dadurch gekennzeichnet, dass** in mindestens einem Zusatzkanal (28) mindestens eine Lichtleiteinrichtung (8) angeordnet ist, wobei im Bereich des ersten Schlauchendes (22) an der mindestens einen Lichtleiteinrichtung (8) mindestens eine Lichteintrittseinrichtung (81) ausgestaltet ist und wobei die mindestens eine Lichtleiteinrichtung (8) am zweiten Schlauchende (23) durch das Abschlusselement (26) zu dem schlauchabgewandten Ende (261) verläuft, wo mindestens eine Lichtaustrittseinrichtung (82) angeordnet ist.

10. Medizinisches System (6) nach Anspruch 8 oder 9, **dadurch gekennzeichnet, dass** in mindestens einem Zusatzkanal (28) eine Seilzugeinrichtung (14) gegenüber der Schlauchwandung (24) verschieblich geführt ist, wobei die Seilzugeinrichtung (14) am zweiten Schlauchende (23), bevorzugt am Abschlusselement (26), befestigt ist und im Bereich des ersten Schlauchendes (22) über den mindestens einem Zusatzkanal (28) aus der Schlauchwandung (24) herausgeführt ist.

11. Medizinisches System (6) nach Anspruch 10, **dadurch gekennzeichnet, dass** in dem mindestens einen, die Seilzugeinrichtung (14) führenden, Zusatzkanal (28) entlang der Schlauchlängsachse (21) mehrere Seilzugführungen (141) beabstandet angeordnet sind.

12. Medizinisches System (6) nach Anspruch 11, **dadurch gekennzeichnet, dass** die Seilzugführungen (141) an dem Zusatzkanal (28) befestigt oder einstückig mit diesem ausgeführt sind.

13. Medizinisches System (6) nach Anspruch 10 bis 12, **dadurch gekennzeichnet, dass** im Bereich des ersten Schlauchendes (22) eine Einrichtung (142) zur Verkürzung des innerhalb des mindestens einen Zusatzkanals (28) geführten Anteils der Seilzugeinrichtung (14) angeordnet ist.

14. Medizinisches System (6) nach einem der Ansprüche 6 bis 13, **dadurch gekennzeichnet, dass** die ringförmige Ausnehmung (93) von dem zweiten Ende (5) in Richtung des ersten Endes (3) sich kontinuierlich verjüngt, wobei die Abmessungen der Ausnehmung (93) an die durchschnittliche Wanddicke (t) der an die zweite Anschlusseinrichtung (9) anzukoppelnden medizinischen Gerätes (4) angepasst ist.

15. Medizinisches System (6) nach einem der Ansprüche 6 bis 14, **dadurch gekennzeichnet, dass** die Abmessungen der ersten Vertiefung (71) an einen Außendurchmesser (D) des anzukoppelnden gastrischen Kalibrierschlauches (2) angepasst sind.

## Claims

1. Connector device (1) for gastric calibration hoses (2) comprising a first and an opposite second end (3, 5), wherein a first connector means (7) is formed at the first end (3) and a second connector means (9) is formed at the second end (5), wherein the first and the second connector means (7, 9) are connected with each other via a fluid channel (11), and wherein the first connector means (7) comprises at least one first tapering cavity (71) into which gastric calibration hose (2) may be plugged for coupling,
**characterized in that**
for coupling different medical devices (4), the second connector means (9) has at least one second tapering cavity (91) and at least one, tapering recess (93) annularly surrounding the second cavity (91), so that, for coupling, the different medical devices (4) may selectively be plugged into the second tapering cavity (91) or into the tapering recess (93),
wherein a delimiting wall (95) and an outer wall (97) surrounding the recess (93) are formed between the second cavity (91) and the recess (93), wherein the outer wall (97) is designed to be set back towards the second end (5) with respect to the delimiting wall (95), such that the delimiting wall (95) forms the first end (3),
wherein the delimiting wall (95) is chamfered in the region of the second end (5) with respect to the longitudinal axis (13).

2. Connector device (1) of claim 1, **characterized in that** the first cavity (71) tapers from the first end (3) towards the second end (5) and the second cavity (91) tapers from the second end (5) towards the first end (3).

3. Connector device (1) of claim 1 or 2, **characterized in that** the first tapering cavity (71) and/or the second tapering cavity (91) form a part of the fluid channel (11).

4. Connector device (1) of claim 3, **characterized in that** an outer diameter (99) of the delimiting wall (95) decreases from the first end (3) towards the second end (5).

5. Connector device (1) of one of claims 1 to 4, **characterized in that**, from the first end (3) towards the second end (5), the first cavity (71) comprises at least two contiguous, respectively tapering steps (73) for receiving gastric calibration hoses (2) with different average diameters (75m), wherein the steps (73) have different initial and end diameters (75a, 75e), and wherein the respective average diameter (75) of the steps (73) respectively decreases continuously from the first end (3) towards the second end (5).

6. Medical system (6) comprising a connector device (1) of one of claims 1 to 5, as well as a gastric calibration hose (2), wherein the gastric calibration hose (2) comprises:
- at least a hose longitudinal axis (21),
- a first and an opposite second hose end (22, 23), and
- a hose wall (24) that encloses a lumen (25),
- wherein an end element (26) is arranged at the second end (26), delimiting the lumen (25), wherein the end element (26) comprises at least one passage (27) extending through the end element (26) in a manner substantially parallel to the hose longitudinal axis (21),
wherein the first hose end (22) is coupled to the connector device (1) via the first connector means (7).

7. Medical system (6) of claim 6, **characterized in that** the hose wall (24) has a plurality of openings (241) in the region of the second hose end (23).

8. Medical system (6) of claim 6 or 7, **characterized in that** the gastric calibration hose (2) comprises at least one auxiliary channel (28), wherein the at least one auxiliary channel (28) encloses a volume (281) and extends substantially parallel to the hose longitudinal axis (21), wherein a second end (283) of the at least one auxiliary channel (28) opens into the end element (26).

9. Medical system (6) of claim 8, **characterized in that** a light conductor means (8) is provided in at least one auxiliary channel (28), wherein a light entry surface (81) is formed at the light conductor device (8) in the region of the first hose end (22), and wherein, at the second hose end (23), the at least one light conductor device (8) extends through the end element (26) to the end (261) averted from the hose, where at least one light emission means (82) is arranged.

10. Medical system (6) of claim 8 or 9, **characterized in that**, in at least one auxiliary channel (28), a cable pull means (14) is guided for sliding movement with respect to the hose wall (24), wherein the cable pull means (14) is fastened at the second hose end (22), preferably at the end element (26), and, in the region of the first hose end (22), is guided out from the hose wall (24) via the at least one auxiliary channel (28).

11. Medical system (6) of claim 10, **characterized in that** in the at least one auxiliary channel (28) guiding the cable pull means (14), a plurality of cable pull guides (141) is arranged along the hose longitudinal axis (21), spaced from each other.

12. Medical system (6) of claim 11, **characterized in that** the cable pull guides (141) are fastened to the auxiliary channel (28) or are formed integrally with the same.

13. Medical system (6) of claims 10 to 12, **characterized in that** a means (142) for shortening the part of the cable pull means (14) guided in the at least one auxiliary channel (28) is arranged in the region of the first hose end (22).

14. Medical system (6) of one of claims 6 to 13, **characterized in that** the annular recess (93) continuously tapers from the second end (5) towards the first end (3), wherein the dimensions of the recess (93) are adapted to the average wall thickness (t) of the medical devices (4) to be coupled to the second connector means (9).

15. Medical system (6) of one of claims 6 to 14, **characterized in that** the dimensions of the first cavity (71) are adapted to an outer diameter (D) of the gastric calibration hose (2) to be coupled thereto.

## Revendications

1. Dispositif de connexion (1) pour sondes de calibrage (2) gastriques comportant une première extrémité et une seconde extrémité opposée (3, 5), dans lequel un premier moyen de connexion (7) est réalisé au niveau de la première extrémité (3) et un second moyen de connexion (9) est réalisé au niveau de la seconde extrémité (5), dans lequel le premier et le second moyen de connexion (7, 9) sont raccordés l'un à l'autre par l'intermédiaire d'un canal pour fluide (11) et dans lequel le premier moyen de connexion (7) présente au moins un premier renfoncement (71) se rétrécissant dans lequel peuvent être enfichées des sondes de calibrage (2) gastriques pour le couplage,
**caractérisé en ce que**
le second moyen de connexion (9) présente, pour le couplage de différents appareils médicaux (4), au moins un second renfoncement (91) se rétrécissant et au moins un évidement (93) se rétrécissant et entourant en forme d'anneau le second renfoncement (91), de sorte que les différents appareils médicaux (4) peuvent être enfichés pour le couplage, au choix, dans le second renfoncement (91) se rétrécissant ou dans l'évidement (93) se rétrécissant,
dans lequel une paroi de délimitation (95) et une paroi extérieure (97) entourant l'évidement (93) sont réalisées entre le second renfoncement (91) et l'évidement (93), dans lequel la paroi extérieure (97) est conçue en retrait par rapport à la paroi de délimitation (95) en direction de la première extrémité (3) de telle sorte que la paroi de délimitation (95) forme la seconde extrémité (5),
dans lequel la paroi de délimitation (95) est mise en œuvre en biseau par rapport à l'axe longitudinal (13) dans la zone de la seconde extrémité (5).

2. Dispositif de connexion (1) selon la revendication 1,
**caractérisé en ce que** le premier renfoncement (71) se rétrécit à partir de la première extrémité (3) en direction de la seconde extrémité (5) et le second renfoncement (91) se rétrécit à partir de la seconde extrémité (5) en direction de la première extrémité (3).

3. Dispositif de connexion (1) selon la revendication 1 ou 2,
**caractérisé en ce que** le premier renfoncement (71) se rétrécissant et/ou le second renfoncement (91) se rétrécissant forment une partie du canal pour fluide (11).

4. Dispositif de connexion (1) selon la revendication 3, **caractérisé en ce qu'**un diamètre extérieur (99) de la paroi de délimitation (95) diminue à partir de la première extrémité (3) en direction de la seconde extrémité (5).

5. Dispositif de connexion (1) selon l'une des revendications 1 à 4,
**caractérisé en ce que** le premier renfoncement (71) comprend, à partir de la première extrémité (3) en direction de la seconde extrémité (5), au moins deux paliers (73) se reliant l'un à l'autre et se rétrécissant respectivement pour permettre la réception des sondes de calibrage (2) gastriques comportant différents diamètres moyens (75m), dans lequel les paliers (73) présentent différents diamètres de début et de fin (75a, 75e) et dans lequel le diamètre moyen (75m) respectif des paliers (73) se réduit respectivement de manière continue à partir de la première extrémité (3) en direction de la seconde extrémité (5).

6. Système médical (6) comprenant un dispositif de connexion (1) selon l'une des revendications 1 à 5, ainsi qu'une sonde de calibrage (2) gastrique, dans lequel la sonde de calibrage (2) présente
- au moins un axe longitudinal de sonde (21),
- une première extrémité de sonde et une seconde extrémité de sonde (22, 23) opposée, et
- une paroi de sonde (24) qui encercle une cavité (25),
- dans lequel un élément de terminaison (26) délimitant la cavité (25) est disposé au niveau de la seconde extrémité de sonde (23), dans lequel l'élément de terminaison (26) présente au moins un passage (27) s'étendant à travers l'élément de terminaison (26) et évoluant sensiblement parallèlement à l'axe longitudinal de sonde (21),
dans lequel la première extrémité de sonde (22) est couplée au dispositif de connexion (1) par l'intermédiaire du premier moyen de connexion (7).

7. Système médical (6) selon la revendication 6, **caractérisé en ce que** la paroi de sonde (24) comprend plusieurs ouvertures (241) dans la zone de la seconde extrémité de sonde (23).

8. Système médical (6) selon la revendication 6 ou 7, **caractérisé en ce que** la sonde de calibrage (2) comprend au moins un canal supplémentaire (28), dans lequel l'au moins un canal supplémentaire (28) encercle un volume (281) et s'étend sensiblement parallèlement à l'axe longitudinal de sonde (21), dans lequel une seconde extrémité (283) de l'au moins un canal supplémentaire (28) débouche dans l'élément de terminaison (26).

9. Système médical (6) selon la revendication 8, **caractérisé en ce qu'**au moins un moyen formant guide de lumière (8) est disposé dans au moins un canal supplémentaire (28), dans lequel au moins un moyen d'entrée de lumière (81) est conçu dans la zone de la première extrémité de sonde (22) sur l'au moins un moyen formant guide de lumière (8) et dans lequel l'au moins un moyen formant guide de lumière (8) s'étend à partir de la seconde extrémité de sonde (23) à travers l'élément de terminaison (26) vers l'extrémité (261) opposée à la sonde où est disposé au moins un moyen de sortie de lumière (82).

10. Système médical (6) selon la revendication 8 ou 9,
**caractérisé en ce que,** dans au moins un canal supplémentaire (28), un moyen de traction de câble (14) est guidé de manière coulissante par rapport à la paroi de sonde (24), dans lequel le moyen de traction de câble (14) est fixé à la seconde extrémité de sonde (23), de préférence à l'élément de terminaison (26), et est guidé hors de la paroi de sonde (24) dans la zone de la première extrémité de sonde (22) par l'intermédiaire de l'au moins un canal supplémentaire (28).

11. Système médical (6) selon la revendication 10, **caractérisé en ce que** plusieurs guides de traction de câble (141) sont disposés à distance le long de l'axe longitudinal de sonde (21) dans l'au moins un canal supplémentaire (28) guidant le moyen de traction de câble (14).

12. Système médical (6) selon la revendication 11, **caractérisé en ce que** les guides de traction de câble (141) sont fixés au canal supplémentaire (28) ou sont mis en œuvre d'un seul tenant avec celui-ci.

13. Système médical (6) selon les revendications 10 à 12, **caractérisé en ce qu'**un moyen (142) permettant de raccourcir la portion du moyen de traction de câble (14) qui est guidée à l'intérieur de l'au moins un canal supplémentaire (28) est disposé dans la zone de la première extrémité de sonde (22).

14. Système médical (6) selon l'une des revendications 6 à 13,
**caractérisé en ce que** l'évidement (93) annulaire se rétrécit de manière continue à partir de la seconde extrémité (5) en direction de la première extrémité (3), dans lequel les dimensions de l'évidement (93) sont adaptées à l'épaisseur de paroi moyenne (t) de l'appareil médical (4) à coupler au second moyen de connexion (9).

15. Système médical (6) selon l'une des revendications 6 à 14,
**caractérisé en ce que** les dimensions du premier renfoncement (71) sont adaptées à un diamètre extérieur (D) de la sonde de calibrage (2) gastrique à coupler.
